# EUROPÄISCHE PATENTSCHRIFT

(11) **EP 1 708 627 B1**
(45) Veröffentlichungstag und Bekanntmachung des Hinweises auf die Patenterteilung: **08.01.2014**
(21) Anmeldenummer: 05701105.8
(22) Anmeldetag: 21.01.2005
(51) Int. Cl.: A61B 17/12

(54) **VORRICHTUNG ZUR IMPLANTATION VON ELEKTRISCH ISOLIERTEN OKKLUSIONSWENDELN**
DEVICE FOR IMPLANTING ELECTRICALLY ISOLATED OCCLUSION HELIXES
DISPOSITIF D'IMPLANTATION D'HELICES D'OCCLUSION ELECTRIQUEMENT ISOLEES

(30) Priorität: 21.01.2004 DE 102004003265
(43) Veröffentlichungstag der Anmeldung: 11.10.2006
(73) Patentinhaber: Dendron GmbH, 44799 Bochum (DE)
(72) Erfinder: MONSTADT, Hermann, 44797 Bochum (DE)
(74) Vertreter: HOFFMANN EITLE
(86) Internationale Anmeldenummer: PCT/EP2005/000587
(87) Internationale Veröffentlichungsnummer: WO 2005/070308

(56) Entgegenhaltungen:
- DE-A1- 10 118 017
- US-A- 5 941 888
- US-A1- 2002 151 883
- US-B1- 6 468 266

## Beschreibung

Die Erfindung betrifft eine Vorrichtung zur Implantation von durch Elektrolyse ablösbaren Okklusionswendeln in Blutgefäßen und Körperhohlräumen, insbesondere Aneurysmen, mit einer Einführhilfe, wenigstens einer distal zur Einführhilfe angeordneten Okklusionswendel und wenigstens einem elektrolytisch korrodierbar ausgebildeten Ablöseelement, wobei zwischen Ablöseelement und Okklusionswendel wenigstens eine Stabilisierungswendel angeordnet ist.

Der Einsatz endovaskulärer Techniken zur Okklusion von Körperhohlräumen oder Gefäßen wie Arterien, Venen, Eileitern oder vaskulären Fehlbildungen (z. B. vaskulärer Aneurysmen) ist bekannter Stand der Technik. Die Okklusionswendel wird dabei in der Regel mit Hilfe eines als Einführhilfe dienenden endovaskulären Führungsdrahtes durch einen Katheter in den zu okkludierenden Hohlraum eingeführt und dort deponiert.

Zur Abtrennung der Okklusionswendel von der Einführhilfe sind aus dem Stand der Technik verschiedene Verfahren bekannt. Besonders bewährt hat sich dabei die elektrolytische Ablösung von Edelstahl-Drahtspitzen, wie sie erstmalig bei der Elektrokoagulation durch Thompson et al. sowie McAlister et al. im Jahre 1979 beschrieben wurde (Radiology 133:335-340, Nov. 1979; AJR 132:998-1000, Juni 1979).

Darauf aufbauend beschreibt auch die EP 0 484 468 eine Vorrichtung zur Implantation von Okklusionswendeln, basierend auf der elektrolytisch korrodierbaren Ausbildung der Drahtspitze des Führungsdrahtes an der Verbindung zwischen Führungsdraht und Okklusionswendel. Auf diese Weise wird die zur Elektrothrombosierung an die als Anode dienende Okklusionswendel angelegte elektrische Spannung für die gleichzeitige Abtrennung der Drahtspitze und somit die Freisetzung der Okklusionswendel genutzt.

Bei derartigen Vorrichtungen macht sich nachteilig bemerkbar, daß der Führungsdraht zur sicheren Führung des Implantates vergleichsweise massiv ausgebildet werden muß, weshalb die Abtrennung der Führungsdrahtspitze mittels Elektrolyse vergleichsweise lange Zeiträume in Anspruch nimmt. Aus disem Grund schlägt die WO 03/017852 A1 vor, eine elektrolytisch korrodierbare Stelle in Form eines Ablöseelements als Teil der Okklusionswendel selbst auszubilden. Da diese Ablöseelemente während des Implantationsprozesses einer erheblich geringeren Biegebeanspruchung unterliegen, können sie einen entsprechend geringeren Durchmesser aufweisen, was zu einer verbesserten und schnelleren elektrolytischen Ablösbarkeit der Okklusionswendel führt. Darüber hinaus wurde es auf diese Weise möglich, für das Ablöseelement weniger stabile, flexiblere Werkstoffe einzusetzen, wodurch ebenfalls die Ablösbarkeit mittels Elektrolyse beschleunigt und verbessert wurde.

Aus dem Stand der Technik ist darüber hinaus die Verwendung von Stabilisierungswendeln bekannt, welche die Ablöseelemente mit den Okklusionswendeln selbst verbinden. Diese Stabilisierungswendeln dienen der Stabilisierung und Versteifung des Implantates und weisen einen geringeren Durchmesser auf als die Okklusionswendeln, so daß sie zumindest teilweise in letztere eingeführt werden können. Darüber hinaus dienen die mit den Ablöseelementen verbundenen Stabilisierungswendeln dazu, die Oberfläche am Ende des Ablöseelementes zu vergrößern, um auf diese Weise eine bessere Verbindbarkeit zur Okklusionswendel zu gewährleisten. Üblicherweise erfolgt die Herstellung der Verbindung mit Hilfe eines Laserschweißverfahrens, wobei die direkte Verbindung eines Ablöseelementes ohne Stabilisierungswendel mit der Okklusionswendel aufgrund des geringen Durchmessers des Ablöseelements ausgesprochen schwierig wäre. Die Herstellung der Verbindung hat sich durch die zusätzliche Verbindung einer Stabilisierungswendel mit entsprechend größerer Oberfläche zwar vereinfacht, dennoch bleibt die Laserschweißtechnik sehr diffizil und aufwendig, so daß hier Bedarf nach einer einfacheren Möglichkeit zur Herstellung der Verbindung von Stabilisierungs- und Okklusionswendel besteht.

Um die Ablösezeiten möglichst gering zu halten, ist es darüber hinaus aus dem Stand der Technik, beispielsweise aus der US 6 620 152 und US 6 468 266 bekannt, das Implantat elektrisch zu isolieren, wodurch sich offenbar die Anfälligkeit des für die elektrolytische Ablösung vorgesehenen Abschnittes des Drahtes für eine elektrolytische Korrosion erhöht. Die Isolierung kann dabei entweder durch Vorsehen einer Isolierung zwischen Implantat und Ablösestelle oder aber durch Überziehen des Implantates mit einer Isolationsschicht vorgenommen werden.

Nachteilig macht sich jedoch bei diesem Stand der Technik bemerkbar, daß sich die Ablösestelle bei der in der genannten Druckschrift beschriebenen Vorrichtung im Führungsdraht befindet, was aus den oben ausgeführten und in der WO 03/017852 A1, auf welche hiermit ausdrücklich Bezug genommen wird, ausführlich beschriebenen Gründen im Vergleich zur Verlagerung der Ablösestelle in den Bereich der Okklusionswendel weniger vorteilhaft ist.

Darüber hinaus sind bei den genannten isolierten Implantaten aus dem Stand der Technik keine zusätzlichen Stabilisierungswendeln vorgesehen, welche der Positionierung und Verbesserung des Übergangs zur Okklusionswendel dienen.

Ausgehend von diesem Stand der Technik stellt sich daher die Aufgabe, eine Vorrichtung zur Implantation von Okklusionswendeln zu schaffen, bei der die für die elektrolytische Korrosion benötigte Zeit weiter herabgesetzt ist und bei der darüber hinaus die Verbindung von Stabilisierungswendel und Okklusionswendel vereinfacht ist.

Diese Aufgabe wird erfindungsgemäß gelöst durch eine Vorrichtung zur Implantation von durch Elektrolyse ablösbaren Okklusionswendeln in Blutgefäßen und Körperhohlräumen, insbesondere Aneurysmen, mit einer Einführhilfe, wenigstens einer distal zur Einführhilfe angeordneten Okklusionswendel und wenigstens einem elektrolytisch korrodierbar ausgebildeten Ablöseelement, wobei zwischen Ablöseelement und Okklusionswendel wenigstens eine Stabilisierungswendel angeordnet ist und wobei die Stabilisierungswendel über eine elektrisch isolierende Klebeschicht mit der Okklusionswendel verbunden ist, so daß die Okklusionswendel bei Anlegen einer elektrischen Spannung an das Ablöseelement von der Spannung isoliert ist.

Im Gegensatz zu den Isolierungen aus dem Stand der Technik befindet sich die Isolierung gemäß der Erfindung nicht zwischen Ablösestelle und Implantat, sondern zwischen einzelnen Bestandteilen des Implantates. Auf diese Weise werden gleich zwei Probleme in einem Schritt gelöst, nämlich die Vereinfachung der Verbindung von Stabilisierungs- und Okklusionswendel als auch die Verkürzung der Ablösezeit. Letzteres ist darauf zurückzuführen, daß sich bei Anlegen einer Spannung an das Ablöseelement die Stromdichte hierin erhöht, wenn andere Teile des Implantates, an denen keine elektrolytische Korrosion stattfinden soll, von der angelegten Spannung isoliert sind.

Gleichzeitig ist die Herstellung der Verbindung zwischen Stabilisierungs- und Okklusionswendel im Vergleich zum Stand der Technik deutlich vereinfacht, wo in der Regel ein Laserschweißverfahren zum Einsatz kommt. Erfindungsgemäß wird dabei üblicherweise so vorgegangen, daß die Stabilisierungswendel mit einem Klebstoff an zumindest dem für die Verbindung mit der Okklusionswendel vorgesehenen Ende versehen und anschließend an die Okklusionswendel angesetzt wird. Besonders vorteilhaft ist es dabei, die Stabilisierungswendel im Ganzen mit einem elektrisch isolierenden Überzug zu versehen, wobei es sich insbesondere um eine Klebebeschichtung handeln kann. Anschließend kann die Stabilisierungswendel an einem Ende an die Okklusionswendel angesetzt oder teilweise in diese eingeführt werden, um eine dauerhafte, isolierende Verbindung herzustellen. Durch die zusätzliche Isolierung der Stabilisierungswendeln mit Hilfe einer elektrisch isolierenden Beschichtung wird jegliche Korrosion im Bereich der Stabilisierungswendel verhindert und die Stromdichte im Bereich des Ablöseelements weiter erhöht, was mit einer entsprechenden Verringerung der Ablösezeit verbunden ist. Gegebenenfalls können auch mehrere ineinandergefügte Stabilisierungswendeln an der Okklusionswendel angesetzt werden.

Zusätzlich ist es zweckmäßig, daß durch das Lumen der Okklusionswendel ein Sicherungsmittel verläuft. Derartige Sicherungsmittel haben den Vorteil, daß im Falle einer fehlerhaften Positionierung der Okklusionswendel oder einer für den zu okkludierenden Bereich zu groß dimensionierten Okklusionswendel das notwendige Zurückziehen der Okklusionswendel, gegebenenfalls vollständig in den Katheter, deutlich sicherer wird. Das Zurückziehen einer Okklusionswendel ohne Sicherungsmittel birgt die Gefahr, daß Teile der Wendel durch Zug- oder Torsionsbeanspruchung auseinandergezogen und so irreversibel plastisch deformiert werden. Im Extremfall kann die Wendel reißen oder brechen, was eine lebensgefährliche Embolie nach sich ziehen kann.

Es ist bekannt, derartige Sicherungsmittel als flexible Polymerfäden auszubilden. Besonders bevorzugt ist jedoch eine Variante, wie sie in der DE 101 18 017 A1 beschrieben wird, derzufolge es sich bei dem Sicherungsmittel um einen Draht handelt, welcher aus einem Material, insbesondere einem Metall mit Formgedächtniseigenschaften gefertigt ist. Ein solches metallisches Sicherungsmittel weist im Vergleich zu Sicherungsmitteln, die aus einem Polymermaterial gefertigt sind, deutliche Vorteile hinsichtlich der Torsions- oder der Zugbelastung auf. Ein aus einem Formgedächtnismetall hergestelltes Sicherungsmittel kann darüber hinaus über eine auch als "Superelastizität" bezeichnete Eigenschaft verfügen, weshalb ein solches Sicherungsmittel in seinem flexiblen Zustand besonders stark durch Biegung oder Zug beansprucht werden kann, ohne daß die Gefahr des Reißens besteht.

Bei den Formgedächtniseigenschaften des Sicherungsmittels kann es sich sowohl um einen thermischen als auch um einen mechanischen Formgedächtniseffekt handeln. Besonders bewährt hat sich hier die Verwendung von Titan und Nickel enthaltenden Legierungen, insbesondere eine dem Fachmann unter dem Namen Nitinol bekannte Legierung. Darüber hinaus sind jedoch Alternativen, z. B. die Verwendung von Eisenbasis- oder Kupferbasislegierungen, möglich. Die genauen Eigenschaften des Formgedächtnismaterial können in für den Fachmann bekannter Weise durch die genaue Wahl der Zusammensetzung gesteuert werden. Der Durchmesser eines solchen, als Sicherungsmittel dienenden Drahtes liegt typischerweise zwischen 0,03 und 0,05 mm.

Darüber hinaus kann die Verwendung eines Formgedächtnismaterials für das Sicherungsmittel genutzt werden, um der Okklusionswendel nach Ausbringung aus dem Katheter in beispielsweise das Aneurysma eine gewünschte übergeordnete Struktur aufzuprägen. Es ist grundsätzlich vorteilhaft, wenn die Okklusionswendel im Aneurysma eine solche Sekundärstruktur ausbildet, beispielsweise helikale Windungen oder eine korbartige Form, da auf diese Weise das Aneurysma besonders effektiv ausgefüllt wird, um eine wirkungsvolle Thrombosierung des Aneurysmas zu gewährleisten. Gegebenenfalls kann hierzu auch die Okklusionswendel selbst zu einer übergeordneten Struktur vorgeformt sein, welche sie nach der Ausbringung aus dem Katheter annimmt. Unter Umständen ist es jedoch ausreichend, wenn lediglich das Sicherungsmittel, nicht aber die Okklusionswendel selbst vorgeformt ist, wenn die vom Sicherungsmittel ausgehende Kraft groß genug ist, die Okklusionswendel ebenfalls in die durch das Sicherungsmittel vorgegebene Form zu zwingen. Die durch das Sicherungsmittel ausgeübte Kraft wird dadurch hervorgerufen, daß das Sicherungsmittel bei Ausbringung in das Aneurysma von dem durch den Katheter ausgeübten Zwang befreit wird und eine Rückumwandlung in seine Austenit-Phase eingeht, wobei es die ihm zuvor aufgeprägte Struktur einnimmt. Zusätzlich oder alternativ kann auch eine temperaturinduzierte Transformation auftreten, wenn das Sicherungsmittel bei Ausschieben aus dem Katheter einer erhöhten Temperatur im Blutstrom ausgesetzt wird.

Das Sicherungsmittel verläuft bei Vorsehen lediglich eines Ablöseelements bzw. bei mehreren Ablöseelementen im Bereich des am weitesten distal gelegenen Segments der Okklusionswendel sinnvollerweise zwischen Stabilisierungswendel und distalem Spitzenabschnitt der Okklusionswendel, d. h. es ist an seinem proximalen Ende mit der Stabilisierungswendel und an seinem distalen Ende mit der Okklusionswendel verbunden. Um zu gewährleisten, daß nicht über das Sicherungsmittel eine elektrische Verbindung zwischen Stabilisierungswendel und Okklusionswendel hergestellt wird, erfolgt die Verbindung von Sicherungsmittel und distalem Spitzenabschnitt der Okklusionswendel zweckmäßigerweise über eine Klebeschicht, welche die Okklusionswendel von einer an das Ablöseelement angelegten elektrischen Spannung isoliert. Die Verbindung von Stabilisierungswendel und metallischem Sicherungsmittel kann, je nach Bedarf, ebenfalls über eine Klebeschicht oder aber mittels Schweißen oder Löten ausgebildet werden. Im Falle der Verwendung einer Klebeschicht ist auch das Sicherungsmittel gegenüber dem Ablöseelement elektrisch isoliert, während bei Schweißen oder Löten die zwecks elektrolytischer Korrosion an das Ablöseelement angelegte Spannung auch am Sicherungsmittel anliegt. Letzteres kann, wie weiter unten beschrieben wird, in bestimmten Fällen vorteilhaft sein.

Um im letzteren Fall zu verhindern, daß bei Kontakt des metallischen Sicherungsmittels mit der Okklusionswendel ein Stromfluß stattfindet, sollte das Sicherungsmittel mit einem elektrisch isolierenden Überzug versehen werden, ähnlich wie er auch für die Stabilisierungswendel eingesetzt werden kann. Ein solcher Überzug ist auch deshalb sinnvoll, weil er die elektrolytische Korrosion des Sicherungsmittels selbst verhindert und die Stromdichte am Ablöseelement hochhält.

Eine Alternative zur elektrischen Isolierung des Sicherungsmittels besteht darin, die Okklusionswendel selbst zumindest an ihrer Innenseite mit einem elektrisch isolierenden Überzug zu versehen. Auch auf diese Weise wird wirkungsvoll verhindert, daß zwischen Sicherungsmittel und Okklusionswendel ein Stromfluß stattfinden kann, sobald sich die beiden Elemente berühren.

Besonders bevorzugt sind solche Vorrichtungen, bei denen die Okklusionswendel über mehrere, voneinander beabstandete, elektrolytisch korrodierbar ausgebildete Ablöseelemente verfügt bzw. bei der mehrere voneinander beabstandete Okklusionswendeln vorgesehen sind, zwischen denen jeweils ein elektrolytisch korrodierbar ausgebildetes Ablöseelement angeordnet ist. Derartige Vorrichtungen sind grundsätzlich aus der WO 01/32085 A1 bekannt, auf welche hiermit Bezug genommen wird. Auf diese Weise können eine oder mehrere variabel dimensionierte Längen der Okklusionswendel durch Elektrolyse abgelöst und im Aneurysma platziert werden. Dies erlaubt es, daß Okklusionswendeln in genau der richtigen Länge in dem Aneurysma abgesetzt werden können. Gegebenenfalls können auch mehrere Längen derselben Wendel nacheinander abgelöst und in den zu okkludierenden Hohlraum eingebracht werden. Dies spart nicht nur Kosten und Zeit, sondern dient insbesondere auch der Minimierung des Operationsrisikos. Darüber hinaus wird auf diese Weise gewährleistet, daß für verschieden große Aneurysmen nicht stets unterschiedlich dimensionierte Okklusionswendeln bereitgehalten und verwendet werden müssen, sondern stattdessen eine einheitliche Vorrichtung verwendet werden kann, bei der je nach Bedarf unterschiedlich große Abschnitte der Okklusionswendel in das Aneurysma eingeführt werden können.

Die Verwendung derartiger, an mehreren Stellen elektrolytisch korrodierbarer Okklusionswendeln beruht auf der Erkenntnis, daß bei Anlegen eines Stroms an eine solche Vorrichtung sich spezifisch die dem distalen Ende des Katheters am nächsten liegende Ablösestelle der Okklusionswendel durch Elektrolyse löst. Dies ist darauf zurückzuführen, daß einerseits die sich im Katheter befindenden elektrolytisch korrodierbaren Stellen durch den Katheter vom ionischen Medium isoliert sind und deshalb keiner Elektrolyse unterliegen können und andererseits die Stromdichte aufgrund des nach distal zunehmenden Widerstandes in der bzw. den Okklusionswendeln von proximal nach distal abnimmt. Die sich nach distal als erstes an das distale Katheterende anschließende elektrolytisch korrodierbar ausgebildete Stelle ist deshalb am stärksten elektrolytischen Prozessen unterworfen und löst sich bevorzugt auf.

Im Falle einer Vorrichtung mit mehreren Ablöseelementen ist es zweckmäßig, daß in den zwischen den Ablöseelementen liegenden Segmenten der Okklusionswendel bzw. den einzelnen Okklusionswendeln jeweils ein Sicherungsmittel angeordnet ist und durch das Lumen der Okklusionswendeln verläuft. Auf diese Weise wird die Deponierung variabel dimensionierbarer Längen verbunden mit einer gleichzeitigen Sicherung jedes einzelnen, zwischen den elektrolytisch korrodierbaren Stellen angeordneten Segmentes und somit ein Höchstmaß an Sicherung gegen das Abreißen von Okklusionswendeln gewährleistet.

Gemäß einer besonders bevorzugten Ausführungsform werden die einzelnen Ablöseelemente elektrisch leitend über metallische Sicherungsmittel verbunden. Gemäß dieser Ausführungsform ist das Sicherungsmittel zwar gegenüber der Okklusionswendel isoliert, leitet jedoch den Strom weiter zum nächsten, distal gelegenen Ablöseelement. Der Übergang kann dabei so erfolgen, daß das Sicherungsmittel direkt mit dem distal nächstgelegenen Ablöseelement verbunden ist oder aber auch in der Form, daß das Sicherungsmittel eine leitende Verbindung mit einer zusätzlichen Stabilisierungswendel am distalen Ende eines Segmentes einer Okklusionswendel bzw. einer einzelnen von mehreren hintereinander angeordneten Okklusionswendeln aufweist, wobei wiederum die Stabilisierungswendel leitend mit dem Ablöseelement verbunden ist. Typischerweise weisen die Ablöseelemente an beiden Enden jeweils eine Stabilisierungswendel auf, die das Ablöseelement mit den entsprechenden Okklusionswendelsegmenten verbinden. Vorzugsweise erstreckt sich dabei jedes einzelne Sicherungsmittel von der Stabilisierungswendel, die sich am distalen Ende eines Ablöseelements befindet, zur distal nächstgelegenen Stabilisierungswendel des folgenden Ablöseeleements. Auf diese Weise wird sichergestellt, daß die einzellen Ablöseelemente über die Stabilisierungswendeln und die hiermit verbundenen Sicherungsmittel elektrisch leitend miteinander verbunden sind, so daß eine zur Ablösung der Okklusionswendel angelegte elektrische Spannung an die einzelnen Ablöseelemente weitergeleitet wird. Gleichzeitig ist jedoch jedes einzelne Okklusionswendelsegment elektrisch gegenüber der Stabilisierungswendel isoliert, da es mit den Stabilisierungswendeln über eine elektrisch isolierende Klebeschicht verbunden ist. Grundsätzlich kann das metallische Sicherungsmittel jedoch auch an den Ablöseelementen selbst festgelegt sein und auf diese Weise den Stromfluß sicherstellen. Lediglich beim am weitesten distal gelegenen Okklusionswendelsegment erstreckt sich das Sicherungsmittel bevorzugt bis zum distalen Spitzenabschnitt der Okklusionswendel selbst, wo das Sicherungsmittel über eine Klebeschicht mit dem distalen Spitzenabschnitt verbunden ist, welche die Okklusionswendel von der zur elektrolytischen Korrosion anlegbaren elektrischen Spannung isoliert.

Zusätzlich wird das Sicherungsmittel vorzugsweise mit einer elektrisch isolierenden Beschichtung überzogen, so daß der elektrische Strom zwar nach distal durch das Innere des Sicherungsmittels weitergeführt wird, in diesem Bereich jedoch keine elektrolytische Korrosion und kein elektrischer Kontakt mit der Okklusionswendel stattfinden kann. Durch die geschilderten Maßnahmen wird eine sehr elegante Möglichkeit zur Schaffung variabel ablösbarer Okklusionswendeln geschaffen und gleichzeitig die Stromdichte am Ablöseelement besonders niedrig gehalten, was mit einer entsprechenden Reduzierung der Ablösezeiten verbunden ist. Darüber hinaus ist diese Ausführungsform auch fertigungstechnisch vorteilhaft, da eine Verbindung über Klebeschichten einfacher herzustellen ist als über die aus dem Stand der Technik bekannten Laserschweißverfahren.

Für die Ausbildung der Klebeschichten und der elektrisch isolierenden Überzüge auf Sicherungsmittel und/oder Stabilisierungswendeln sind grundsätzlich verschiedene Materialien denkbar. Wichtig ist hierbei naturgemäß die Verträglichkeit und möglichst auch die Zulassung im medizinischen Bereich.

Neben der Biokompatibilität und den Isolierungseigenschaften ist auch die Härtbarkeit des Klebers von Vorteil, wobei insbesondere durch Strahlung härtbare Kleber, z. B. UV-härtbare Kleber, vorteilhaft sind, da hier gegebenenfalls durch die zur Härtung einwirkende Strahlung gleichzeitig eine Sterilisation hervorgerufen wird. Zweckmäßig ist insbesondere die Verwendung von Acrylat-Klebstoffen, obgleich grundsätzlich auch andere Kleber mit den angesprochenen Eigenschaften geeignet sind.

Besonders bevorzugt ist dabei der Einsatz eines unter der Bezeichnung "Permabond" vertriebenen Materials, welches zugelassen ist und sich im medizinischen Bereich ausgezeichnet bewährt hat. Permabond 4L25, das von der National Starche & Chemical Company (ICI) vertrieben wird, ist ein UV-härtbarer Klebstoff, der im medizinischen Bereich für die Verbindung einer Vielzahl von Stoffen geeignet ist. Es handelt sich um einen Klebstoff auf Acrylat-Basis, der bei einer Wellenlänge von 320 bis 380 nm gehärtet werden kann. Nach Härtung besteht Permabond USP Klasse VI Tests. Darüber hinaus ist Permabond unempfindlich gegenüber Gammastrahlung und eine Reihe anderer Sterilisationsverfahren.

Die Ablöseelemente, die für eine rasche Korrosion vorgesehen sind, bestehen vorzugsweise aus einer Stahllegierung. Bevorzugt sind hier insbesondere nicht rostende Stähle der Typen AISI 301, 303 oder 316 bzw. Untergruppen dieser Typen. Derartige nicht rostende Stähle haben vorzugsweise einen Chromanteil zwischen 12 und 20 Gew.-%. Beispiele hierfür sind die Stahlqualitäten 1.4410, 1.4310, 1.4301 und 1.4122. Geeignet sind beispielsweise Chrom/Nickelstähle der Qualität 18/8. Der Durchmesser der Ablöseelemente liegt typischerweise zwischen 0,01 und 0,05 mm.

Um die Ablösezeiten weiter zu verkürzen, können die Ablöseelemente vorkorrodiert sein. Eine solche Vorkorrosion kann beispielsweise in Form einer Wärmebehandlung erfolgen, durch die das Metall in seinem Gefüge so verändert wird, daß es sich bei Anlegen einer elektrischen Spannung in einem Elektrolyten besonders rasch zersetzt. Die hierzu erforderliche Wärmebehandlung kann mit Hilfe eines Lasers vorgenommen werden, in einem üblichen Ofen oder mittels einer Induktionsspule. Der dazu erforderliche Temperaturbereich liegt bei etwa 500 bis 1000°C, vorzugsweise bei 600 bis 950°C und insbesondere bei 700 bis 900°C. Es wird angenommen, daß bei dieser Wärmebehandlung eine Rekristallisation unter Ausbildung von großen Gefügekörnern und Hartmetallkarbiden stattfindet und damit eine Verringerung der Korngrenzenstabilität. Die Ausbildung von Chromkarbiden, die sich auf den Korngrenzen abscheiden, führt zu einer Verarmung der Matrix an Chrom sowie zu einer Herabsetzung des Widerstandes gegen interkristalline Korrosion. Die für die Korrosion zur Verfügung stehende Oberfläche wird dadurch rasch erweitert, so daß es zu einer schnellen Zersetzung des Gefüges unter Stromanschluß in einem Elektrolyt kommt.

Eine weitere Möglichkeit, die Ablöseelemente besonders gut korrodierbar auszubilden, besteht darin, in den entsprechenden Bereichen Materialkombinationen zu wählen, die geeignet sind, Lokalelemente auszubilden. Beispiele hierfür sind Kombinationen aus nicht rostenden Stählen mit Edelmetallen bzw. Edelmetalliegierungen, insbesondere Platinlegierungen.

Für die Ausbildung der Okklusionswendeln selbst hat sich die Verwendung von Platin oder Platinlegierungen bewährt. Besonders bevorzugt ist dabei die Verwendung von Platin-Iridiumlegierungen. Die mit den Ablöseelementen verbundenen Stabilisierungswendeln bestehen zumeist, ähnlich wie die Ablöseelemente selbst, aus einer Stahllegierung, grundsätzlich ist jedoch auch für die Stabilisierungswendeln die Verwendung einer Platinlegierung ähnlich der für die Okklusionswendeln verwendeten möglich. Die Verwendung einer Stahllegierung ist insofern vorteilhaft, als auf diese Weise der Röntgenkontrast zwischen Ablöseelement und Stabilisierungswendel einerseits und Okklusionswendel andererseits erhöht wird. Dies ist darauf zurückzuführen, daß Platinlegierungen eine erheblich höhere Röntgendichte aufweisen als Stahllegierungen.

Bei der Einführhilfe handelt es sich vorzugsweise um einen herkömmlichen Führungsdraht, wie er sich bewährt hat, um Okklusionswendeln durch einen Katheter zu einem zu okkludierenden Hohlraum zu führen.

Die erfindungsgemäße Vorrichtung kann auch direkt in Kombination mit einem Mikrokatheter vorliegen, durch den die Okklusionswendel mit Hilfe der Einführhilfe an ihren Bestimmungsort gebracht wird. Der verwendete Katheter und die verwendete Okklusionswendel sollten dabei hinsichtlich ihrer Dimensionen aufeinander abgestimmt sein. Gegebenenfalls kann der Katheter dabei auch einen Zwang auf die Okklusionswendel sowie das Sicherungsmittel ausüben, der bewirkt, daß die Okklusionswendel erst nach Befreiung von dem Zwang eine ihr bzw. dem Sicherungsmittel zuvor aufgeprägte Sekundärstruktur im Aneurysma einnimmt. Zweckmäßigerweise ist der Katheter darüber hinaus mit röntgendichten Markierungen versehen, die die Plazierung im Zielbereich mit Hilfe bekannter Bildgebungsverfahren ermöglicht.

Neben der Vorrichtung zur Implantation von elektrolytisch ablösbaren Okklusionswendeln betrifft die Erfindung auch ein entsprechendes medizinisches Implantat, bestehend aus wenigstens einer Okklusionswendel, wenigstens einem Ablöseelement und wenigstens einer Stabilisierungswendel, wobei die Stabilisierungswendel zwischen Ablöseelement und Okklusionswendel angeordnet ist, um auf diese Weise Ablöseelement und Okklusionswendel indirekt miteinander zu verbinden, und wobei die Stabilisierungswendel über eine elektrisch isolierende Klebeschicht mit der Okklusionswendel verbunden ist.

Die Erfindung wird nachfolgend beispielhaft anhand der beigefügten Figuren näher erläutert. Es zeigen:
- Figur 1: die schematische Darstellung der Positionierung einer Okklusionswendel in einem Beerenaneurysma mit Hilfe der erfindungsgemäßen Vorrichtung;
- Figur 2: einen Längsschnitt durch die erfindungsgemäße Vorrichtung in gegenüber Figur 1 deutlich vergrößerter Darstellung; und
- Figur 3: einen Längsschnitt durch die erfindungsgemäße Vorrichtung mit mehreren Ablöseelementen.

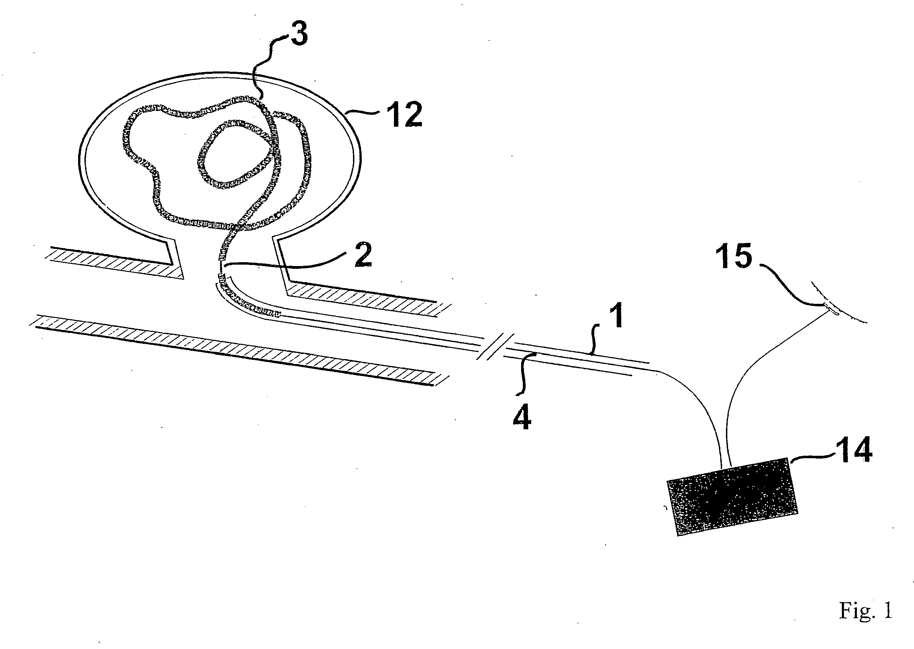

In Figur 1 ist die vertikale Ansicht einer in einem Beerenaneurysma 12 posionierten Okklusionswendel 3 dargestellt. Die Okklusionswendel 3 wird mit Hilfe des Führungsdrahtes 4 innerhalb des Katheters 1 nach distal verschoben. Bei korrekter Positionierung tritt die Okklusionswendel 3 aus dem Ende des Katheters 1 heraus in den durch das Beerenaneurysma 12 gebildeten Hohlraum und füllt diesen aus. Innerhalb des Aneurysmas 12 bildet die Okklusionswendel 3 sekundäre Windungen aus, was insbesondere durch einen spannungsund/oder temperaturinduzierten Übergang der Okklusionswendel 3 und/oder des hier nicht dargestellten Sicherungsmittels im Inneren der Okklusionswendel 3 von der Martensit- in die Austenitphase hervorgerufen werden kann. Aufgrund der Ausbildung von sekundären Windungen wird das Aneurysma 12 besonders effektiv ausgefüllt.

Sobald eine an das Volumen des auszufüllenden Hohlraumes angepaßte Länge der Okklusionswendel 3 in das Aneurysma 12 eingebracht ist, erfolgt die elektrolytische Ablösung am Ablöseelement 2. Hierzu wird mit Hilfe der Spannungsquelle 14 eine elektrische Spannung an das Ablöseelement 2 angelegt, wobei das Ablöseelement 2 als Anode dient. Die Kathode 15 wird auf der Körperoberfläche positioniert. Bei der erfindungsgemäßen Vorrichtung erfolgt die elektrolytische Korrosion des Ablöseelements 2 innerhalb besonders kurzer Zeiten, die in der Regel deutlich unter 1 min, bei ca. 20 bis 40 s (bei 2 V, 2 mA) liegen. Gemäß einer bevorzugten Ausführungsform sind im Bereich der Okklusionswendel 3 mehrere Ablöseelemente 2 vorgesehen, so daß die Länge der eingebrachten Okklusionswendel 3 jeweils individuell an das Aneurysma 12 angepaßt werden kann.

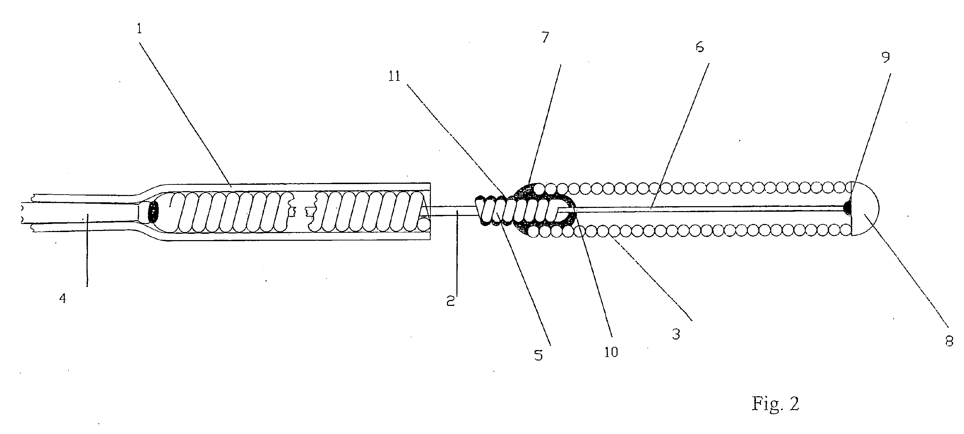

In Figur 2 ist die erfindungsgemäße Vorrichtung schematisch als Längsschnitt dargestellt. Die hier dargestellte Okklusionswendel 3 ist über eine Stabilisierungswendel 5 mit dem Ablöseelement 2 verbunden, wobei es sich bei der Stabilisierungswendel5 um eine Wendel kleineren Durchmessers als die Okklusionswendel 3 selbst handelt und der Außendurchmesser der Stabilisierungswendel 5 im wesentlichen dem Innendurchmesser der Okklusionswendel 3 entspricht. Entsprechend kann die Stabilisierungswendel 5 zumindest teilweise in die Okklusionswendel 3 eingeführt werden.

Stabilisierungswendel 5 und Okklusionswendel 3 sind über eine Klebeschicht 7 miteinander verbunden, wobei die Klebeschicht 7 gleichzeitig für eine Isolierung der Okklusionswendel 3 von der an das Ablöseelement 2 angelegten elektrischen Spannung sorgt. Auf diese Weise wird die Stromdichte im Ablöseelement 2 besonders hoch gehalten. Dieser Effekt wird durch eine zusätzliche Beschichtung 11 auf der Stabilisierungswendel 5 weiter verstärkt. Die Stromzufuhr zwecks Ablösung der Okklusionswendel 3 kann mit Hilfe des Führungsdrahtes 4 zum Ablöseelement 2 hin erfolgen.

Zusätzlich ist im Lumen der Okklusionswendel 3 ein Sicherungsmittel 6 vorgesehen, bei dem es sich um einen Nitinol-Draht handelt, der mit einer isolierenden Beschichtung versehen ist. Das Sicherungsmittel 6 erstreckt sich von der Stabilisierungswendel 5 zum distalen Ende 8 der Okklusionswendel 3. An dieser Stelle wird die Verbindung von Sicherungsmittel 6 und distalem Ende 8 der Okklusionswendel 3 über eine weitere isolierende Klebeschicht 9 hergestellt. Zur Herstellung der Klebeschichten 7 und 8 sowie für die Beschichtung auf der Stabilisierungswendel 5 wird Permabond verwendet.

Zur Herstellung der erfindungsgemäßen Vorrichtung wird zunächst eine Stabilisierungswendel 5 mit einer Permabond-Schicht versehen und anschließend das als Nitinoldraht ausgebildete Sicherungsmittel 6 mit der Stabilisierungswendel 5 an der Stelle 10, d. h. am distalen Ende der Stabilisierungswendel 5, verschweißt. Schließlich wird die Stabilisierungswendel 5 mit Hilfe von Permabond an der Okklusionswendel 3 festgelegt.

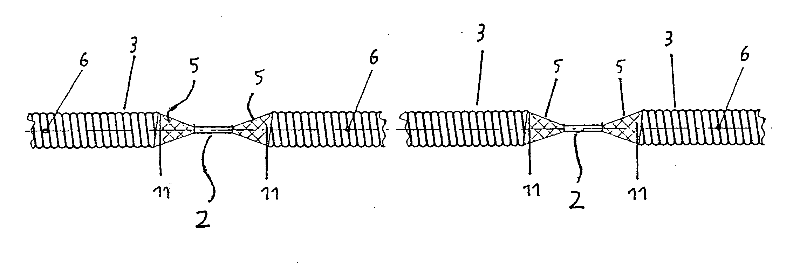

In Figur 3 ist eine erfindungsgemäße Vorrichtung mit mehreren Ablöseelementen 2 gezeigt. Die Vorrichtung verfügt über mehrere Okklusionswendelsegmente 3, wobei die Ablösung je nach Bedarf und Größe des zu verschließenden Aneurysmas an unterschiedlichen Ablöseelementen 2 erfolgen kann. Jedes Ablöseelement 2 ist dabei über eine Stabilisierungswendel 5 mit dem jeweiligen Okklusionswendelsegment 3 verbunden, wobei die Verbindung erfindungsgemäß über eine elektrisch isolierende Klebeschicht hergestellt wird. Zusätzlich ist die Stabilisierungswendel 5 hier mit einer ebenfalls elektrisch isolierenden Beschichtung 11 versehen.

Durch das Lumen der jeweiligen Okklusionswendelsegmente 3 verläuft ein metallisches Sicherungsmittel 6, das mit einem elektrisch isolierenden Überzug versehen ist, um die Isolierung des Sicherungsmittels 6 gegenüber der Okklusionswendel 3 sicherzustellen. Durch das Innere des Sicherungsmittels 6 wird jedoch der elektrische Strom an das entsprechende Ablöseelement 2 nach distal weitergeleitet, um hier bei Bedarf die elektrolytische Korrosion hervorrufen zu können. Innerhalb eines Okklusionswendelsegments 3 erstreckt sich das metallische Sicherungsmittel 6 jeweils von der proximal mit dem Okklusionswendelsegment 3 verbundenen Stabilisierungswendel 5 bis zur distal mit dem Okklusionswendelsegment 3 verbundenen Stabilisierungswendel 5. Auf diese Weise wird der elektrische Strom von einem Ablöseelement 2 über die hiermit verbundene Stabilisierungswendel 5, das Sicherungsmittel 6 zur distal sich anschließenden Stabilisierungswendel 5 und zum damit verbundenen Ablöseelement 2 geleitet. Das Sicherungsmittel 6 wird entsprechend gleich in doppelter Hinsicht vorteilhaft ausgenutzt, da es zum einen der Erhöhung der Sicherheit im Falle einer fehlerhaften Positionierung einer Okklusionswendel 3 und zum anderen der elektrisch leitenden Verbindung der einzelnen Ablöseelemente 2 dient.

## Patentansprüche

1. Vorrichtung zur Implantation von durch Elektrolyse ablösbaren Okklusionswendeln (3) in Blutgefäßen und Körperhohlräumen, insbesondere Aneurysmen (12), mit einer Einführhilfe (4), wenigstens einer distal zur Einführhilfe (4) angeordneten Okklusionswendel (3) und wenigstens einem elektrolytisch korrodierbar ausgebildeten Ablöseelement (2), **dadurch gekennzeichnet, dass** das Ablöseelement (2) im Bereich der Okklusionswendel (3) angeordnet ist, das Ablöseelement (2) über wenigstens eine Stabilisierungswendel (5), die zwischen Ablöseelement (2) und Okklusionswendel (3) angeordnet ist, mit der Okklusionswendel (3) verbunden ist, wobei die Stabilisierungswendel (5) zumindest teilweise in die Okklusionswendel (3) eingeführt ist und die Stabilisierungswendel (5) über eine elektrisch isolierende Klebeschicht (7) mit der Okklusionswendel (3) verbunden ist, so dass die Okklusionswendel (3) bei Anlegen einer elektrischen Spannung an das Ablöseelement (2) von der Spannung isoliert ist.

2. Vorrichtung nach Anspruch 1, **dadurch gekennzeichnet, dass** die Stabilisierungswendel (5) mit einem elektrisch isolierenden Überzug (11) versehen ist.

3. Vorrichtung nach Anspruch 1 oder 2, **dadurch gekennzeichnet, dass** durch das Lumen der Okklusionswendel (3) ein Sicherungsmittel (6) verläuft.

4. Vorrichtung nach Anspruch 3, **dadurch gekennzeichnet, dass** das Sicherungsmittel (6) aus einem Material mit Formgedächtniseigenschaften besteht.

5. Vorrichtung nach Anspruch 4, **dadurch gekennzeichnet, dass** das Sicherungsmittel (6) bei Ausbringung in das Blutgefäß oder den Körperhohlraum eine Transformation eingeht und eine ihm zuvor aufgeprägte Struktur einnimmt.

6. Vorrichtung nach Anspruch 4 oder 5, **dadurch gekennzeichnet, dass** das Sicherungsmittel (6) aus Nitinol besteht.

7. Vorrichtung nach einem der Ansprüche 3 bis 6, **dadurch gekennzeichnet, dass** sich zumindest ein Sicherungsmittel (6) von der Stabilisierungswendel (5) bis zum distalen Spitzenabschnitt (8) der Okklusionswendel (3) erstreckt.

8. Vorrichtung nach Anspruch 7, **dadurch gekennzeichnet, dass** das zumindest eine Sicherungsmittel (6) über eine Klebeschicht (9) mit dem distalen Spitzenabschnitt (8) der Okklusionswendel (3) verbunden ist, welche die Okklusionswendel (3) von einer an das Ablöseelement (2) angelegten elektrischen Spannung isoliert.

9. Vorrichtung nach einem der Ansprüche 3 bis 8, **dadurch gekennzeichnet, dass** das Sicherungsmittel (6) mit einem elektrisch isolierenden Überzug versehen ist.

10. Vorrichtung nach einem der Ansprüche 3 bis 9, **dadurch gekennzeichnet, dass** die Okklusionswendel (3) zumindest an ihrer Innenseite mit einem elektrisch isolierenden Überzug versehen ist.

11. Vorrichtung nach einem der Ansprüche 1 bis 10, **dadurch gekennzeichnet, dass** die Okklusionswendel (3) über mehrere, voneinander beabstandete, elektrolytisch korrodierbar ausgebildete Ablöseelemente (2) verfügt.

12. Vorrichtung nach einem der Ansprüche 1 bis 10, **gekennzeichnet durch** mehrere voneinander beabstandete Okklusionswendeln (3), wobei zwischen den einzelnen Okklusionswendeln (3) jeweils ein elektrolytisch korrodierbar ausgebildetes Ablöseelement (2) angeordnet ist.

13. Vorrichtung nach Anspruch 11 oder 12, **dadurch gekennzeichnet, dass** in den zwischen den Ablöseelementen (2) liegenden Segmenten der Okklusionswendel (3) bzw. den einzelnen Okklusionswendeln (3) jeweils ein Sicherungsmittel (6) angeordnet ist.

14. Vorrichtung nach Anspruch 13, **dadurch gekennzeichnet, dass** sich zumindest einige der Sicherungsmittel (6) jeweils von einer mit einem Ablöseelement (2) verbundenen Stabilisierungswendel (5) zur distal nächstgelegenen Stabilisierungswendel (5) erstrecken.

15. Vorrichtung nach Anspruch 13, **dadurch gekennzeichnet, dass** sich zumindest einige der Sicherungsmittel (6) von einem Ablöseelement (2) zum distal nächstgelegenen Ablöseelement (2) erstrecken.

16. Vorrichtung nach einem der Ansprüche 11 bis 15, **dadurch gekennzeichnet, dass** die Ablöseelemente (2) über die durch das Lumen der Okklusionswendeln (3) verlaufenden Sicherungsmittel (6) elektrisch leitend miteinander verbunden sind.

17. Vorrichtung nach einem der Ansprüche 1 bis 16, **dadurch gekennzeichnet, dass** die Klebeschichten (7, 9) und/oder die elektrisch isolierenden Überzüge (11) aus einem Acrylat-Klebstoff bestehen.

18. Vorrichtung nach Anspruch 17, **dadurch gekennzeichnet, dass** der Acrylat-Klebstoff Permabond ist.

19. Vorrichtung nach einem der Ansprüche 1 bis 18, **dadurch gekennzeichnet, dass** die Ablöseelemente (2) aus einer Stahllegierung bestehen.

20. Vorrichtung nach einem der Ansprüche 1 bis 19, **dadurch gekennzeichnet, dass** die Ablöseelemente (2) vorkorrodiert sind.

21. Vorrichtung nach einem der Ansprüche 1 bis 20, **dadurch gekennzeichnet, dass** die Okklusionswendeln (3) aus Platin oder einer Platinlegierung, insbesondere aus einer Platin-Iridium-Legierung bestehen.

22. Vorrichtung nach einem der Ansprüche 1 bis 21, **dadurch gekennzeichnet, dass** die Einführhilfe ein Führungsdraht (4) ist.

23. Vorrichtung nach einem der Ansprüche 1 bis 22, **dadurch gekennzeichnet, dass** sie in Kombination mit einem Mikrokatheter (1) vorliegt.

24. Medizinisches Implantat zur Verwendung in Verbindung mit einer Vorrichtung nach einem der Ansprüche 1 bis 23, das Implantat, bestehend aus wenigstens einer Okklusionswendel (3), wenigstens einem elektrolytisch korrodierbar ausgebildeten Ablöseelement (2) und wenigstens einer Stabilisierungswendel (5), wobei das Ablöseelement (2) über die Stabilisierungswendel (5), die zwischen Ablöseelement (2) und Okklusionswendel (3) angeordnet ist, mit der Okklusionswendel (3) verbunden ist, wobei die Stabilisierungswendel (5) zumindest teilweise in die Okklusionswendel (3) eingeführt ist und die Stabilisierungswendel (5) über eine elektrisch isolierende Klebeschicht (7) mit der Okklusionswendel (3) verbunden ist, so dass die Okklusionswendel (3) bei Anlegen einer elektrischen Spannung an das Ablöseelement (2) von der Spannung isoliert ist.

## Claims

1. Device for implantation of occlusion coils (3) which can be detached by electrolysis in blood vessels and body cavities, in particular aneurysms (12), with an introduction aid (4), at least one occlusion coil (3) arranged distally to the introduction aid (4) and at least one detachment element (2) designed to be electrolytically corrodible, **characterised in that** the detachment element (2) is arranged in the region of the occlusion coil (3), the detachment element (2) is connected to the occlusion coil (3) via at least one stabilisation coil (5) which is arranged between detachment element (2) and occlusion coil (3), wherein the stabilisation coil (5) is introduced at least partly into the occlusion coil (3) and the stabilisation coil (5) is connected to the occlusion coil (3) via an electrically insulating adhesive layer (7) so that the occlusion coil (3) is insulated from the voltage when applying an electric voltage to the detachment element (2).

2. Device according to claim 1, **characterised in that** the stabilisation coil (5) is provided with an electrically insulating coating (11).

3. Device according to claim 1 or 2, **characterised in that** a securing means (6) runs through the lumen of the occlusion coil (3).

4. Device according to claim 3, **characterised in that** the securing means (6) consists of a material having shape-retention properties.

5. Device according to claim 4, **characterised in that** the securing means (6) enters a transformation when it is placed into the blood vessel or the body cavity and assumes a structure impressed on it previously.

6. Device according to claim 4 or 5, **characterised in that** the securing means (6) consists of Nitinol.

7. Device according to one of claims 3 to 6, **characterised in that** at least one securing means (6) extends from the stabilisation coil (5) up to the distal tip section (8) of the occlusion coil (3).

8. Device according to claim 7, **characterised in that** the at least one securing means (6) is connected via an adhesive layer (9) to the distal tip section (8) of the occlusion coil (3) which insulates the occlusion coil (3) from an electrical voltage applied to the detachment element (2).

9. Device according to one of claims 3 to 8, **characterised in that** the securing means (6) is provided with an electrically insulating coating.

10. Device according to one of claims 3 to 9, **characterised in that** the occlusion coil (3) is provided with an electrically insulating coating at least on its inner side.

11. Device according to one of claims 1 to 10, **characterised in that** the occlusion coil (3) has several detachment elements (2) spaced from one another and designed to be electrolytically corrodible.

12. Device according to one of claims 1 to 10, **characterised by** several occlusion coils (3) spaced from one another, wherein in each case a detachment element (2) designed to be electrolytically corrodible is arranged between the individual occlusion coils (3).

13. Device according to claim 11 or 12, **characterised in that** in each case a securing means (6) is arranged in the segments of the occlusion coil (3) or the individual occlusion coils (3) lying between the detachment elements (2).

14. Device according to claim 13, **characterised in that** at least some of the securing means (6) extend in each case from a stabilisation coil (5) connected to a detachment element (2) to the distally nearest stabilisation coil (5).

15. Device according to claim 13, **characterised in that** at least some of the securing means (6) extend from a detachment element (2) to the distally nearest detachment element (2).

16. Device according to one of claims 11 to 15, **characterised in that** the detachment elements (2) are connected to one another to be electrically conductive via the securing means (6) running through the lumen of the occlusion coils (3).

17. Device according to one of claims 1 to 16, **characterised in that** the adhesive layers (7, 9) and/or the electrically insulating coatings (11) consist of an acrylate adhesive.

18. Device according to claim 17, **characterised in that** the acrylate adhesive is Permabond.

19. Device according to one of claims 1 to 18, **characterised in that** the detachment elements (2) consist of a steel alloy.

20. Device according to one of claims 1 to 19, **characterised in that** the detachment elements (2) are pre-corroded.

21. Device according to one of claims 1 to 20, **characterised in that** the occlusion coils (3) consist of platinum or a platinum alloy, in particular of a platinum-iridium alloy.

22. Device according to one of claims 1 to 21, **characterised in that** the introduction aid is a guide wire (4).

23. Device according to one of claims 1 to 22, **characterised in that** it is present in combination with a microcatheter (1).

24. Medical implant for use in conjunction with a device according to one of claims 1 to 23, the implant consisting of at least one occlusion coil (3), at least one detachment element (2) designed to be electrolytically corrodible and at least one stabilisation coil (5), wherein the detachment element (2) is connected to the occlusion coil (3) via the stabilisation coil (5) which is arranged between detachment element (2) and occlusion coil (3), wherein the stabilisation coil (5) is introduced at least partly into the occlusion coil (3) and the stabilisation coil (5) is connected to the occlusion coil (3) via an electrically insulating adhesive layer (7) so that the occlusion coil (3) is insulated from the voltage when applying an electric voltage to the detachment element (2).

## Revendications

1. Dispositif d'implantation de spirales d'occlusion (3) détachable par électrolyse dans des vaisseaux sanguins et des cavités corporelles, plus particulièrement des anévrismes (12), avec une aide à l'introduction (4), au moins une spirale d'occlusion (3) disposée de manière distale par rapport à l'aide d'à l'introduction (4) et au moins un élément détachable (2) corrodable de manière électrolytique, **caractérisé en ce que** l'élément détachable (2) est disposé au niveau de la spirale d'occlusion (3), l'élément détachable (2) est relié à la spirale d'occlusion (3) à l'aide d'au moins une spirale de stabilisation (5) qui se trouve entre l'élément détachable (2) et la spirale d'occlusion (3), la spirale de stabilisation (5) étant introduite au moins partiellement dans la spirale d'occlusion (3) et la spirale de stabilisation (5) étant reliée à la spirale d'occlusion (3) à l'aide d'une couche adhésive (7) électriquement isolante, de façon à ce que la spirale d'occlusion (3) soit isolée de la tension lors de l'application d'une tension électrique à l'élément détachable (2).

2. Dispositif selon la revendication 1, **caractérisé en ce que** la spirale de stabilisation (5) est munie d'un revêtement (11) électriquement isolant.

3. Dispositif selon la revendication 1 ou 2, **caractérisé en ce qu'**un moyen de fixation (6) travers le lumen de la spirale d'occlusion (3).

4. Dispositif selon la revendication 3, **caractérisé en ce que** le moyen de fixation (6) est constitué d'un matériau avec des propriétés de mémoire de forme.

5. Dispositif selon la revendication 4, **caractérisé en ce que** le moyen de fixation (6) subit une transformation lors d'une pose dans le vaisseau sanguin ou la cavité corporelle et adopte une structure prédéterminée.

6. Dispositif selon la revendication 4 ou 5, **caractérisé en ce que** le moyen de fixation (6) est constitué de nitinol.

7. Dispositif selon l'une des revendications 3 à 6, **caractérisé en ce qu'**au moins un moyen de fixation (6) s'étend de la spirale de stabilisation (5) jusqu'à la partie de pointe distale (8) de la spirale d'occlusion (3).

8. Dispositif selon la revendications 7, **caractérisé en ce que** l'au moins un moyen de fixation (6) est relié à la partie de pointe distale (8) de la spirale d'occlusion (3) à l'aide d'une couche adhésive (9) qui isole la spirale d'occlusion (3) d'une tension électrique appliquée à l'élément détachable (2).

9. Dispositif selon l'une des revendications 3 à 8, **caractérisé en ce que** le moyen de fixation (6) est muni d'un revêtement électriquement isolant.

10. Dispositif selon l'une des revendications 3 à 9, **caractérisé en ce que** la spirale d'occlusion (3) est munie, au moins sur son côté interne, d'un revêtement électriquement isolant.

11. Dispositif selon l'une des revendications 1 à 10, **caractérisé en ce que** la spirale d'occlusion (3) est munie de plusieurs éléments détachables (2) éloignés les uns des autres et corrodables de manière électrolytique.

12. Dispositif selon l'une des revendications 1 à 10, **caractérisé par** plusieurs spirales d'occlusion (3) éloignés les uns des autres, un élément détachable (2) corrodable de manière électrolytique étant disposé à chaque fois entre les différentes spirales d'occlusion (3).

13. Dispositif selon la revendication 11 ou 12, **caractérisé en ce que**, dans les segments de la spirale d'occlusion (3) ou les différentes spirales d'occlusion (3) se trouvant entre les éléments détachables (2), se trouve à chaque fois un moyen de fixation (6).

14. Dispositif selon la revendication 13, **caractérisé en ce qu'**au moins certains des moyens de fixation (6) s'étendent à chaque fois d'une spirale de stabilisation (5) reliée à un élément détachable (2) vers la spirale de stabilisation (5) distale suivante.

15. Dispositif selon la revendication 13, **caractérisé en ce qu'**au moins certains des moyens de fixation (6) s'étendent d'un élément détachable (2) vers l'élément détachable (2) distal suivant.

16. Dispositif selon l'une des revendications 11 à 15, **caractérisé en ce que** les éléments détachables (2) sont reliés entre eux de manière électro-conductrice à l'aide des moyens de fixation (6) traversant le lumen des spirales d'occlusion (3).

17. Dispositif selon l'une des revendications 1 à 16, **caractérisé en ce que** les couches adhésives (7, 9) et/ou les revêtements (11) électriquement isolants sont constitués d'une colle acrylate.

18. Dispositif selon la revendication 17, **caractérisé en ce que** la colle acrylate est du Permabond.

19. Dispositif selon l'une des revendications 1 à 18, **caractérisé en ce que** les éléments détachables (2) sont constitués d'un alliage d'acier.

20. Dispositif selon l'une des revendications 1 à 19, **caractérisé en ce que** les éléments détachables (2) sont pré-corrodés.

21. Dispositif selon l'une des revendications 1 à 20, **caractérisé en ce que** les spirales d'occlusion (3) sont constituées de platine ou d'un alliage de platine, plus particulièrement d'un alliage platine-iridium.

22. Dispositif selon l'une des revendications 1 à 21, **caractérisé en ce que** l'aide à l'introduction est un fil de guidage (4).

23. Dispositif selon l'une des revendications 1 à 22, **caractérisé en ce qu'**il est utilisé en combinaison avec un micro-cathéter (1).

24. Implant médical destiné à être utilisé en combinaison avec un dispositif selon l'une des revendications 1 à 23, l'implant étant constitué d'au moins une spirale d'occlusion (3), d'au moins un élément détachable (2) corrodable de manière électrolytique et d'au moins une spirale de stabilisation (5), l'élément détachable (2) étant relié à la spirale d'occlusion (3) à l'aide de la spirale de stabilisation (5) qui se trouve entre l'élément détachable (2) et la spirale d'occlusion (3), la spirale de stabilisation (5) étant au moins partiellement introduite dans la spirale d'occlusion (3) et la spirale de stabilisation (5) étant reliée à la spirale d'occlusion (3) à l'aide d'une couche adhésive (7) électriquement isolante, de façon à ce que la spirale d'occlusion (3) est isolée de la tension lors de l'application d'une tension électrique à l'élément détachable (2).
